(19) Europäisches Patentamt
European Patent Office
Office européen des brevets

(11) Publication number: **0 265 400 B1**

# (12) EUROPEAN PATENT SPECIFICATION

(45) Date of publication of patent specification: **12.06.91**

(51) Int. Cl.5: **A61K 47/30**, A61K 47/38, A61K 37/34, A61K 37/02, A61K 31/135

(21) Application number: **87850244.2**

(22) Date of filing: **14.08.87**

## (54) Topical radioprotective gel for mucosa.

(30) Priority: **18.08.86 SE 8603481**

(43) Date of publication of application:
**27.04.88 Bulletin 88/17**

(45) Publication of the grant of the patent:
**12.06.91 Bulletin 91/24**

(84) Designated Contracting States:
**AT BE CH DE ES FR GB IT LI NL SE**

(56) References cited:
**BE-A- 667 456**
**DE-A- 3 106 619**
**FR-A- 2 355 510**
**US-A- 2 785 103**

**NATURE, vol. 205, 23rd January 1965, pages 364-365, MacMillan (Journals) Ltd, London, GB; B. LARSSON et al.: "Reduction of radiation damage to the intestinal mucous membrane by local hypoxia"**

(73) Proprietor: **Ferring AB**
**Soldattorpsvägen 5 Box 30561**
**S-200 62 Malmö(SE)**

(72) Inventor: **Aronsen, Karl Frederik**
**Östanväg 62**
**S-216 19 Malmö(SE)**
Inventor: **Nylander, Göran**
**Notariegatan 19**
**S-216 11 Malmö(SE)**
Inventor: **Borgström, Stig**
**Bodilsgatan 4 B**
**S-217 74 Malmö(SE)**

(74) Representative: **Nilsson, Brita Linnea et al**
**OSCAR GRAHN PATENTBYRA AB, Box 19540**
**S-104 32 Stockholm(SE)**

EP 0 265 400 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 84, no. 3, 19th January 1976, page 70, no. 12832v, Columbus, Ohio, US; A.T. RAPPLEYE et al.: "Radioprotective effects of vasopressin on the gastrointestinal tract of mice", & RADIOLOGY 1975, 117(1), 199-203

CHEMICAL ABSTRACTS, vol. 93, no. 13, 29th September 1980, page 112, no. 126217q, Columbus, Ohio, US; M.C. HAN et al.: "An experimental study on modification of radiation effects by various drugs. I. On radioprotective effects of vasoconstrictors" & INKAN KWAHAK, 1980, 4(5), 348-55

## Description

The present invention relates to a topical radioprotective gel for mucosa and comprises a vasoconstrictive substance, a pharmaceutically acceptable thickening agent, and water.

Radiation therapy of tumours frequently causes secondary effects in the form of inflammation of the adjoining mucosa. Especially patients undergoing radiation therapy in the small pelvic region frequently acquire inflammation of the mucosa in the rectum and colon sigmoideum. Depending on the radiation dose and the length of the treatment, the inflammation may lead to fibrosis and obstruction of the bowel.

Since the beginning of the 1900's it has been known (G. Schwarz, Münch. med. Wochschr., 56, 1217 (1909)) that tissue hypoxia gives a radioprotective effect. Since then, different ways of inducing tissue hypoxia have been investigated, and these investigations were particularly concerned with the temporary mechanical blocking of mesenterial circulation by means of degradable starch microspheres, or with the selective inhibition of mucosal circulation by means of pharmacological drugs. Among the latter, vasopressin, epinephrine, and lysine-vasopressin have so far been tested intravenously or intra-arterially in animals (Rappleye et al, Radiology 117, 199-203 (1975), Steckel et al, Radiology 92, 1341 (1969), S. Borgström et al, British Journal of Radiology 55, 568-573 (1982) and S. Borgström et al, Acta Radiological Oncology 24 (1985), Fasc. 5) in order to protect the gastrointestinal tract, the kidneys or the mucosa of the small bowel. Furthermore, norepinephrine together with sodium sulphite has been tested locally in the rectum of rats by drop infusion (B. Larsson and S. Sténson, Nature, Vol. 205, pp. 364-365 (1965)) in order to protect the rectal mucosa.

US patent 2,785,103 discloses a vasoconstrictive composition l-desoxyephedrine or salts thereof and a pharmaceutical carrier, e.g. adapted for use as an inhalant, but radioprotection of the nasal mucosa is not disclosed.

In the radiation treatment of tumours, the radiation treatment schedule varies, but up to 30 consecutive treatment days is not unusual, and radiation damage may show already after few days. Secondary effects from the bowel are often therapy-limiting in the radiation treatment of the small pelvic region.

The disadvantages encountered in the intra-arterial administration of vasoconstrictive substances in order to induce hypoxia in the mucosa are on the one hand systemic effects, such as blood pressure increase and bradycardia, and, on the other hand, that the patient must have the injection in direct connection with the radiation treatment since the time for the treatment proper is critical because of the short maximum hypoxic effect of certain drugs and because the radiation treatment possibly must take place before the drug has had time to affect the blood supply to the tumour. In addition, intra-arterial administration requires specially trained personnel, for which reason intra-arterial administration here will hardly become a routine technique.

There thus is need for a relatively long-term radioprotective drug against mucosa which is readily administered and which, furthermore, has no generic circulatoric effects.

It has now surprisingly been found that vasoconstrictive substances can be administered topically to mucosa and provide the desired hypoxic effect if administered in gel form.

The present invention comprises a topical radioprotective gel for mucosa, which eliminates the disadvantages encountered in intra-arterial administration of vasoconstrictive substances.

The topical radioprotective gel according to the invention is the first topically applicable gel for the radioprotection of mucosa.

The topical radioprotective gel according to the invention comprises one or more vasoconstrictive substances, selected among triglycyl-lysine-vasopressin, lysine-vasopressin, arginine-vasopressin, ornithine-vasopressin, and phenylalanine-lysine-vasopressin, a pharmaceutically acceptable thickening agent, and water.

The thickening agent should be inert, pharmaceutically acceptable and have adequate stability. The thickening agent should impart to the formulation appropriate rheological properties, such as

- keeping the active substance at the application site for an appropriate time, i.e. the apparent viscosity of the formulation at low stress should be higher than for water;
- a flexible formulation which is not irreversibly altered by the administration process, i.e. when exposed to higher stress.

Preferably, use is made of pharmaceutically acceptable thickeners possessing a shear thinning behaviour (i.e. imparting to the formulation an apparent viscosity which is a diminishing function of the applied stress). For instance, such formulations may be either pseudo-plastic or thixotropic in nature. Soluble polymers, such as carboxypolymethylene, derivatives of cellulose (e.g. carboxymethyl cellulose, hydroxyethyl cellulose, hydroxypropyl cellulose), natural gums (e.g. xanthan, guar) or derivatives of alginic acid (e.g. sodium alginate), will provide a formulation with shear thinning properties (pseudo-plastic). The

use of insoluble hydrocolloids, such as microcrystalline cellulose, will impart to the formulation a thixotropic behaviour. Also mixtures of the above-mentioned types of thickeners can be used to achieve appropriate rheological behaviour. The water used in the gel preferably is sterile deionised water.

Naturally, the radioprotective gel according to the invention may also include conventional pharmaceutical, inert additives, such as stabilisers, salts and preservatives according to the the US and European Pharmacopoeias.

By "gel" is meant, in the context of the present invention, a liquid which is more viscous than water to facilitate its retention on the mucosa during radiation treatment.

The topical radioprotective gel according to the invention may be applied e.g. rectally about 15 min. before radiation treatment of the small pelvic region. To be sure, the pharmacological effect need not be present for more than a few seconds, but the point of time of the radiation treatment is no longer critical because of the gel according to the invention yields the vasoconstrictive substance during a relatively long period of time.

The characteristic features of the radioprotective gel according to the invention will appear from the appended claims.

The vasoconstrictive substances preferred at present are vasopressin derivatives having the following established structural formulae:

Triglycin-lysine-vasopressin (TGLVP)

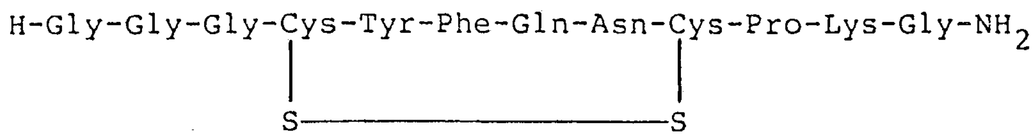

Lysine-vasopressin (LVP)

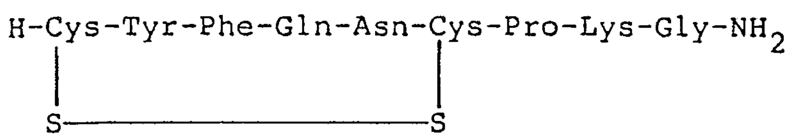

Arginine-vasopressin (AVP)

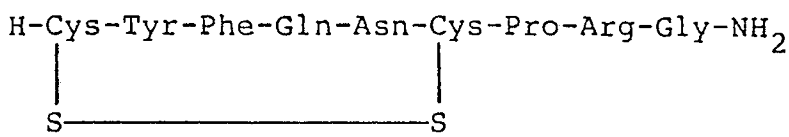

Other suitable vasoconstrictive vasopressin derivatives are:

Ornithine-vasopressin

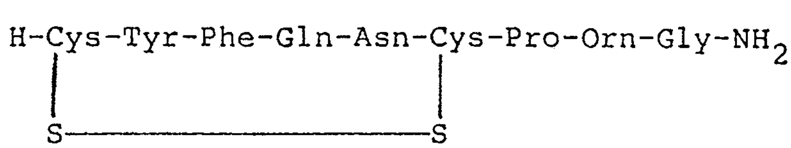

Phenylalanine-lysine-vasopressin

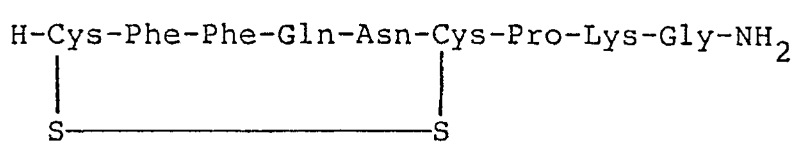

The amounts of the various ingredients included in the topical radioprotective gel are not critical, and the expert will have no difficulty in testing out suitable amounts that give the desired properties which are adequate hypoxic effect, stability and sterility as well as a suitable viscosity to facilitate maintaining the gel in contact with the mucosa during a suitable period of time. Naturally, the amount of the vasoconstrictive substance depends upon which substance is used in a specific case in order to give a suitable hypoxic effect, and this applies also to the thickening agent employed to provide an aqueous solution thereof having suitable viscosity properties and a suitable release of the vasoconstrictive substance or, where appropriate, a mixture of substances.

The concentration of vasoconstrictive substance in the radioprotective gels according to the invention was determined according to the desire to provide a local vasoconstrictive effect (with subsequent hypoxia) in the mucosa, but without general secondary effect, such as blood pressure increase and bradycardia. To achieve this, dose titration studies of the different vasoconstrictive substances were carried out, i.e. the dose just below the threshold of general circulatory response was defined. Due to interaction (electrostatic binding) between the active substance and the different thickening agents, the dose for a specific vasoconstrictive substance varied depending upon the thickening agent employed.

Examples of topical radioprotective gels that have been found to function as intended in radioprotective experiments, are 1600-6400 µg TGLVP per g of 1% by weight carboxymethyl cellulose in sterile deionised water, 10 µg AVP per g of 0.2% by weight carboxypolymethylene in sterile deionised water, and 32 µg LVP per g of 1.2% by weight hydroxyethyl cellulose in sterile deionised water.

To show the effect of radioprotective gels for the mucosa on topical application, a number of tests were carried out.

For studies of systematic circulation, Wistar rats (body weight 177-296 g) were used. TGLVP was administered rectally by means of a Nelaton-Foley catheter inserted through the anus until the balloon was 5 cm away from the anus. The balloon was then filled to ensure that the catheter stayed in position and to prevent TGLVP from reaching the upper parts of the bowel. TGLVP was dissolved in a 1% by weight aqueous solution of sodium carboxymethyl cellulose (CMC) and was in contact with all parts of the mucosa during the test. The volume applied amounted to half a milliliter. Three dose levels of TGLVP were tested, i.e. 800 µg, 1600 µg and 3200 µg. For each dose level, use was made of five rats and a corresponding number of controls which were given CMC solution only. For anaesthesia, nembutal was used intraperitoneally. Mean values for arterial pressure and puls rate were recorded by means of a catheter in the aorta via the femoral artery.

The results of these tests show that the doses 800 µg and 1600 µg gave no systemic circulatory response in the form of blood pressure increase or lower pulse. 3200 µg, on the other hand, caused blood pressure increase in several animals.

Dose titration was carried out in a similar manner on AVP, LVP, TGLVP with other thickening agents.

The following results were obtained.

25 µg and 50 µg AVP were given in 0.2% by weight carboxypolymethylene solution without any significant influence on blood pressure (pulse rate was not measured). LVP was given in 0.2% by weight carboxypolymethylene solution in a dose of 10 µg and 20 µg without any significant change in blood pressure (pulse rate was not measured). The dose of 40 µg and 80 µg gave a rise in blood pressure (40 µg significant at 10, 15, 20, 25, 45 and 60 minutes after application, 80 µg significant 5-60 minutes after application).

LVP in 1% by weight sodiumcarboxymethyl cellulose (CMC) solution was given in a dose of 10 µg, 20 µg and 40 µg without any change in blood pressure (pulse rate was not measured). 80 µg LVP caused a rise in blood pressure 5-45 minutes after application.

LVP in 1.2% by weight hydroxyethyl cellulose solution was given in a dose of 4 µg, 8 µg and 16 µg without significant changes in systemic circulation. 32 µg caused a rise in blood pressure 5, 10, l5, 20, 25, 30, 45, 75 and 90 minutes after application. No decrease in pulse rate was noted.

TGLVP in 0.9% by weight hydroxypropyl cellulose solution was given in doses of 128 µg and 256 µg without changes. 512 µg gave a bradycardia 15-120 minutes after application. With said solution there was a notable tendency to rise in blood pressure in the control group.

TGLVP was also given in 1.2% by weight hydroxyethyl cellulose solution. The dose of 128 µg gave no circulatory changes. 256 µg gave a rise in blood pressure at 75 and 90 minutes after application. There was also a decrease in pulse rate at 5 minutes after application. 512 µg gave a rise in blood pressure 30 and 45 minutes after application and a bradycardia 10-45 minutes after.

For studies of the radioprotective effect of TGLVP Wistar rats (body weight 187-394 g) were used and treated in pairs at the same time. In each pair, one rat was treated rectally with TGLVP, while the other rat, the control rat, was given CMC solution rectally. The gels were applied in the same manner as described

above. For anaesthesia, nembutal was given intraperitoneally. The rats were irradiated with 240 kV X-rays (0.2 Cu filtration), using AP and PA fields in size 4 × 3 cm. This gave a uniform dose in the lower part of the abdomen corresponding to the position of the rectum. 10 Gy were given on day 1, and 10 Gy on day 4. The rats were killed 14 days after the treatment, and the rectum was removed for histological analysis. The rats were weighed before treatment and when they were killed.

10 rats were treated with 1600 µg TGLVP 15 min before irradiation (Tables IV and V)

5 rats were treated with 800 µg TGLVP 15 min before irradiation (Table V)

10 rats were given 1600 µg TGLVP 30 min before treatment (Table III)

5 rats were given 3200 µg TGLVP 15 min before treatment (Table I)

5 rats were given 3200 µg TGLVP 30 min before treatment (Table II)

Histological grading (sum of parameters) was performed in accordance with an evaluation system in which 0 is the normal structure and 6 indicates maximum change. Irradiation-induced lesions in the mucosa were determined arbitrarily by means of the following parameters:

1: The extent of fibrosis in the mucosa and submucosa.

2: The change of glandular structure in the mucosa.

3: Ulcerations in the mucosa or abscesses in mucosal crypts.

The results obtained are shown in Tables I-V. Table VI shows a corresponding radioprotective test with AVP, in which only histological grading was performed.

Table VII concerns a corresponding radioprotection test with LVP.

TABLE 1

15 min

TGLVP 6400 μg/g 1 weight% carboxymethyl cellulose in water

| Rat No. | 1 | 2 | 3 | 4 | 5 | 6 | 7 | 8 | 9 | 10 |
|---|---|---|---|---|---|---|---|---|---|---|
| Dose (μg) | - | 3200 | - | 3200 | - | 3200 | - | 3200 | - | 3200 |
| Fibrosis | 4 | 2 | 4 | 2 | 4 | 2 | 6 | 2 | 4 | 4 |
| Change of glandular structure | 6 | 2 | 6 | 0 | 6 | 0 | 6 | 2 | 6 | 4 |
| Ulceration - abscessa development in crypts | 4 | 2 | 4 | 0 | 4 | 0 | 4 | 0 | 6 | 4 |
| Sum of parameters | 14 | 6 | 14 | 2 | 14 | 2 | 16 | 4 | 16 | 12 |
| Body weight change (g) | 5 | 4 | 14 | 7 | 5 | 15 | 0 | 12 | 4 | 9 |

TABLE II

30 min

TGLVP 6400 µg/g 1 weight% carboxymethyl cellulose in water

| Rat No. | 11 | 12 | 13 | 14 | 15 | 16 | 17 | 18 | 19 | 20 |
|---|---|---|---|---|---|---|---|---|---|---|
| Dose (µg) | - | 3200 | - | 3200 | - | 3200 | - | 3200 | - | 3200 |
| Fibrosis | 6 | 4 | 6 | 4 | 6 | 4 | 6 | 4 | 6 | 4 |
| Change of glandular structure | 6 | 4 | 6 | 0 | 4 | 2 | 6 | 2 | 6 | 0 |
| Ulceration - abscessa development in crypts | 6 | 4 | 6 | 2 | 4 | 0 | 4 | 2 | 6 | 0 |
| Sum of parameters | 18 | 12 | 18 | 6 | 14 | 6 | 16 | 8 | 18 | 4 |
| Body weight change (g) | 24 | 7 | 46 | 13 | 0 | -2 | 9 | 38 | 22 | 24 |

TABLE III

30 min

TGLVP 3200 μg/g 1 weight% carboxymethyl cellulose in water

| Rat No. | 21 | 22 | 23 | 24 | 25 | 26 | 27 | 28 | 29 | 30 |
|---|---|---|---|---|---|---|---|---|---|---|
| Dose (μg) | - | 1600 | - | 1600 | - | 1600 | - | 1600 | - | 1600 |
| Fibrosis | 6 | 4 | 4 | 2 | 6 | 4 | 6 | 4 | 6 | 4 |
| Change of glandular structure | 6 | 2 | 4 | 2 | 6 | 2 | 6 | 4 | 6 | 2 |
| Ulceration - abscessa development in crypts | 6 | 0 | 2 | 2 | 6 | 0 | 6 | 2 | 4 | 0 |
| Sum of parameters | 18 | 6 | 10 | 6 | 18 | 6 | 18 | 10 | 16 | 6 |
| Body weight change (g) | -2 | 3 | 9 | 18 | -4 | 7 | -7 | 5 | 0 | 14 |

TABLE III (cont'd)

| Rat No. | 31 | 32 | 33 | 34 | 35 | 36 | 37 | 38 | 39 | 40 |
|---|---|---|---|---|---|---|---|---|---|---|
| Dose (μg) | - | 1600 | - | 1600 | - | 1600 | - | 1600 | - | 1600 |
| Fibrosis | 6 | 4 | 4 | 2 | 4 | 2 | 4 | 4 | 6 | 2 |
| Change of glandular structure | 6 | 2 | 6 | 2 | 4 | 2 | 6 | 2 | 6 | 2 |
| Ulceration - abscessa development in crypts | 6 | 0 | 6 | 2 | 6 | 2 | 4 | 4 | 6 | 0 |
| Sum of parameters | 18 | 6 | 16 | 6 | 14 | 6 | 14 | 10 | 18 | 4 |
| Body weight change (g) | 11 | 1 | 38 | 66 | 61 | 80 | 39 | 47 | 7 | 55 |

TABLE IV

15 min

TGLVP in 1 weight% carboxymethyl cellulose in water

| Rat No. | 41 | 42 | 43 | 44 | 45 | 46 | 47 | 48 | 49 | 50 |
|---|---|---|---|---|---|---|---|---|---|---|
| Dose (µg) | - | 1600 | - | 1600 | - | 1600 | - | 1600 | - | 1600 |
| Fibrosis | 6 | 2 | 6 | 4 | 4 | 2 | 4 | 2 | 6 | 2 |
| Change of glandular structure | 6 | 2 | 4 | 2 | 4 | 2 | 4 | 2 | 4 | 2 |
| Ulceration - abscessa development in crypts | 4 | 2 | 4 | 0 | 4 | 0 | 4 | 0 | 4 | 0 |
| Sum of parameters | 16 | 6 | 14 | 6 | 12 | 4 | 12 | 4 | 14 | 4 |
| Body weight change (g) | 5 | 11 | -7 | 20 | 1 | 3 | 4 | 3 | 7 | 3 |

TABLE V

15 min

TGLVP in 1 weight% carboxymethyl cellulose in water

| Rat No. | 51 | 52 | 53 | 54 | 55 | 56 | 57 | 58 | 59 |
|---|---|---|---|---|---|---|---|---|---|
| Dose (µg) | - | 800 | 1600 | - | 800 | 1600 | - | 800 | 1600 |
| Fibrosis | 5 | 2 | 1 | 2 | 2 | 2 | 4 | 3 | 3 |
| Change of glandular structure | 5 | 1 | 0 | 3 | 1 | 0 | 4 | 2 | 2 |
| Ulceration - abscessa development in crypts | 5 | 1 | 0 | 2 | 0 | 0 | 4 | 2 | 1 |
| Sum of parameters | 15 | 4 | 1 | 7 | 3 | 2 | 12 | 7 | 6 |
| Body weight change (g) | -5 | -7 | 15 | 2 | 3 | 20 | ? | -14 | 11 |

TABLE V (cont'd)

| Rat No. | 60 | 61 | 62 | 63 | 64 | 65 |
|---|---|---|---|---|---|---|
| Dose (µg) | - | 800 | 1600 | - | 800 | 1600 |
| Fibrosis | 2 | 1 | 2 | 4 | 2 | 3 |
| Change of glandular structure | 2 | 1 | 1 | 3 | 2 | 1 |
| Ulceration - abscessa development in crypts | 2 | 1 | 0 | 3 | 3 | 1 |
| Sum of parameters | 8 | 3 | 3 | 10 | 7 | 5 |
| Body weight change (g) | ? | -3 | 9 | 1 | ? | 14 |

TABLE VI

10 min

20 µg AVP, 0.2 weight% carboxymethylene in water, 1 ml

| Rat No. | 66 | 67 | 68 | 69 | 70 | 71 | 72 | 73 |
|---|---|---|---|---|---|---|---|---|
| AVP (10 µg) | no | yes | no | yes | no | yes | no | yes |
| Histological grading | 5 | 2 | 4 | 5 | 4 | 2 | 4 | 1 |

TABLE VII

15 min

LVP 32 µg/g 1.2 weight% hydroxyethyl cellulose in water

| Rat No. | 74 | 75 | 76 | 77 | 78 | 79 | 80 | 81 | 82 | 83 | 84 | 85 | 86 | 87 | 88 | 89 |
|---|---|---|---|---|---|---|---|---|---|---|---|---|---|---|---|---|
| Dose (µg) | - | 16 | - | 16 | - | 16 | - | 16 | - | 16 | - | 16 | - | 16 | - | 16 |
| Fibrosis | 6 | 2 | 4 | 4 | 6 | 4 | 4 | 4 | 6 | 6 | 6 | 4 | 6 | 2 | 6 | 4 |
| Change of glandular structure | 4 | 0 | 4 | 2 | 6 | 6 | 6 | 4 | 6 | 6 | 6 | 4 | 6 | 2 | 6 | 2 |
| Ulceration - abscessa development in crypts | 4 | 0 | 4 | 4 | 6 | 6 | 6 | 4 | 4 | 6 | 6 | 4 | 4 | 0 | 6 | 2 |
| Sum of parameters | 14 | 2 | 12 | 10 | 18 | 16 | 16 | 12 | 16 | 18 | 18 | 12 | 16 | 4 | 18 | 8 |

Tables I-V above, show that a dose of 3200, 1600 or 800 µg TGLVP upon irradiation for 15 or 30 min after administration gave radioprotection to the mucosa as compared with the controls. Moreover, it appears from Tables I-IV that an increase of the dose from 1600 µg to 3200 µg, or an increase of the time from 15 to 30 min, does not seem to give a better result than 1600 µg at 15 min.

Increasing the dose from 800 µg to 1600 µg gave an improved protection, as will appear from the body

weight values shown in Table V.

A dose of 1600 µg TGLVP thus gives maximum radioprotective effect to rats without general circulatory responses.

Table VI indicates that a dose of 10 µg AVP upon irradiation 10 min after administration gave radioprotection to the mucosa, as compared with the controls.

Table VII indicates that a dose of 16 µg LVP upon irradiation 15 min after administration gave radioprotection to the mucosa and caused no significant change in systemic circulation.

## Claims

1. A topical radioprotective gel for the mucosa, **characterised** in that it comprises one or more vasoconstrictive substances selected among triglycyl-lysine-vasopressin, lysine-vasopressin, arginine-vasopressin, ornithine-vasopressin, and phenylalanine-lysine-vasopressin, a pharmaceutically acceptable thickening agent and water.

2. A topical radioprotective gel as claimed in claim 1, wherein the pharmaceutically acceptable thickening agent is selected among carboxymethyl cellulose, hydroxyethyl cellulose, hydroxypropyl cellulose, and carboxypolymethylene.

Claims for the following Contracting States : AT, ES

1. A method of producing a topical radioprotective gel for the mucosa, **characterised,** by mixing one or more vasoconstrictive substances selected among triglycyl-lysine-vasopressin, lysine-vasopressin, arginine-vasopressin, ornithine-vasopressin, and phenylalanine-lysine-vasopressin, a pharmaceutically acceptable thickening agent and water.

2. A method as claimed in claim 1, wherein the pharmaceutically acceptable thickening agent is selected among carboxymethyl cellulose, hydroxyethyl cellulose, hydroxypropyl cellulose, and carboxypolymethylene.

## Revendications

1. Gel topique radio-protectif pour muqueuses, **caractérisé** par le fait qu'il comprend un ou plusieurs vasoconstricteurs choisis parmi le groupe comportant les triglycyl-lysine-vasopressine, lysine-vasopressine, arginine-vasopressine, ornithine-vasopressine et phénylalanine-lysine-vasopressine, un épaississant pharmaceutiquement acceptable et l'eau.

2. Gel topique radio-protectif selon la revendication 1, dans lequel l'épaississant pharmaceutiquement acceptable est choisi parmi le groupe comportant les cellulose carboxyméthyl, cellulose hydroxyéthyl, cellulose hydroxypropyle et carboxypolyméthylène.

Revendications pour les Etats contractants suivants: AT, ES

1. Procédé de préparation d'un gel topique radioprotectif pour muqueuses, **caractérisé** par le fait que l'on mélange un ou plusieurs vasoconstricteurs choisis parmi le groupe comportant les triglycyl-lysine-vasopressine, lysine-vasopressine, arginine-vasopressine, ornithine-vasopressine et phénylalanine-lysine-vasopressine, un épaississant pharmaceutiquement acceptable et l'eau.

2. Procédé selon la revendication 1, dont lequel l'épaississant pharmaceutiquement acceptable est choisi parmi le groupe comportant les cellulose carboxyméthyl, cellulose hydroxyéthyl, cellulose hydroxypropyle et carboxypolyméthylène.

## Ansprüche

1. Topisches Gel zum Schutz von Schleimhäuten bei Bestrahlungen, dadurch **gekennzeichnet,** dass es einen oder mehrere gefässverengernde Stoffe, gewählt aus der Gruppe, die aus Triglycyl-Lysin-Vasopressin, Lysin-Vasopressin, Arginin-Vasopressin, Ornithin-Vasopressin und Phenyl-Alanin-Lysin-Vasopressin besteht, ein pharmazeutisch akzeptables Verdickungsmittel und Wasser enthält.

2. Topisches Gel zum Schutz bei Bestrahlungen nach Anspruch 1, dadurch **gekennzeichnet,** dass das pharmazeutisch akzeptable Verdickungsmittel aus der Gruppe gewählt ist, die aus Carboxymethylcellulose, Hydroxyäthylcellulose, Hydroxypropylcellulose und Carboxypolymethylen besteht.

Patentansprüche für folgende Vertragsstaaten : AT, ES

1. Verfahren zur Herstellung eines topischen Gels zum Schutz von Schleimhäuten bei Bestrahlungen, **gekennzeichnet** durch Mischen von einem oder mehreren gefässverengernden Stoffen, gewählt aus der Gruppe, die aus Triglycyl-Lysin-Vasopressin, Lysin-Vasopressin, Arginin-Vasopressin, Ornithin-Vasopressin und Phenyl-Alanin-Lysin-Vasopressin besteht, einem pharmazeutisch akzeptablen Verdikkungsmittel und Wasser.

2. Verfahren nach Anspruch 1, dadurch **gekennzeichnet,** dass das pharmazeutisch akzeptable Verdikkungsmittel aus der Gruppe gewählt ist, die aus Carboxymethylcellulose, Hydroxyäthylcellulose, Hydroxypropylcellulose und Carboxypolymethylen besteht.